# EUROPÄISCHE PATENTSCHRIFT

(11) **EP 3 482 764 B1**
(45) Veröffentlichungstag und Bekanntmachung des Hinweises auf die Patenterteilung: **17.03.2021**
(21) Anmeldenummer: 17200805.4
(22) Anmeldetag: 09.11.2017
(51) Int. Cl.: A61K 36/71, A61P 39/00, A61Q 17/02, A61P 17/00

(54) **INSEKTEN- UND/ODER SPINNENTIER-REPELLENT ENTHALTEND SCHWARZKÜMMELÖL**
INSECT AND/OR SPIDER REPELLENT COMPRISING BLACK CUMIN OIL
RÉPULSIF INSECTES ET/OU ARACHNIDES COMPRENANT DE L'HUILE DE NIGELLE

(43) Veröffentlichungstag der Anmeldung: 15.05.2019
(73) Patentinhaber: Anton Hübner GmbH & Co. KG, 79238 Ehrenkirchen (DE)
(72) Erfinder: Gallrein, Andreas, 79254 Oberried (DE); Paulus, Sarah, 79100 Freiburg (DE); Beate, Engelbrecht, 79238 Ehrenkirchen (DE); Dr. Gerecke, Hagen, 04155 Leipzig (DE)
(74) Vertreter: Becker & Kurig Partnerschaft Patentanwälte PartmbB

(56) Entgegenhaltungen:
- DE-U1- 20 209 721
- CARROLL J F ET AL: "Repellency to ticks (Acari: Ixodidae) of extracts ofNigella sativa(Ranunculaceae) and the anti-inflammatory DogsBestFriend(TM)", EXPERIMENTAL AND APPLIED ACAROLOGY, CHAPMAN & HALL, GB, Bd. 70, Nr. 1, 9. Juli 2016 (2016-07-09), Seiten 89-97, XP036032963, ISSN: 0168-8162, DOI: 10.1007/S10493-016-0058-X [gefunden am 2016-07-09]
- NERIO L S ET AL: "Repellent activity of essential oils: A review", BIORESOURCE TECHNOLOGY, ELSEVIER, AMSTERDAM, NL, Bd. 101, Nr. 1, 1. Januar 2010 (2010-01-01), Seiten 372-378, XP026624017, ISSN: 0960-8524, DOI: 10.1016/J.BIORTECH.2009.07.048 [gefunden am 2009-09-02]
- DATABASE BIOSIS [Online] BIOSCIENCES INFORMATION SERVICE, PHILADELPHIA, PA, US; Juni 2007 (2007-06), UPADHYAY RAVIKANT ET AL: "Toxicity, repellency and oviposition inhibitory actwity of some essential oils against Callosobruchus chinensis", XP002780892, Database accession no. PREV200700506920 & JOURNAL OF APPLIED BIOSCIENCE, Bd. 33, Nr. 1, Juni 2007 (2007-06), Seiten 23-28, ISSN: 0379-8097
- PANDEY S K ET AL: "Insecticidal and repellent activities of thymol from the essential oil of Trachyspermum ammi (Linn) Sprague seeds against Anopheles stephensi", PARASITOLOGY RESEARCH ; FOUNDED AS ZEITSCHRIFT FÜR PARASITENKUNDE, SPRINGER, BERLIN, DE, Bd. 105, Nr. 2, 3. April 2009 (2009-04-03) , Seiten 507-512, XP019716288, ISSN: 1432-1955
- HAMMER M L A ET AL: "Tapping an Amazonian plethora: four medicinal plants of Marajo island, Para (Brazil)", JOURNAL OF ETHNOPHARMACOLOGY, ELSEVIER IRELAND LTD, IE, Bd. 40, Nr. 1, 1. September 1993 (1993-09-01), Seiten 53-75, XP025813290, ISSN: 0378-8741, DOI: 10.1016/0378-8741(93)90089-N [gefunden am 1993-09-01]
- Anonymous: "Regensburger Schüler entdeckt wirksames Mittel gegen Zecken", , 30 May 2014 (2014-05-30), XP055612237, Retrieved from the Internet: URL:http://wissenschaftler.de/news/item/49 75-regensburger-schüler-entdeckt-wirksames -mittel-gegen-zecken?tmpl=component&print= 1&jjj=1565602088630 [retrieved on 2019-08-12]

## Beschreibung

Die vorliegende Erfindung betrifft das Gebiet der Repellentien gegen Insekten (Insecta) und/oder gegen Spinnentiere (Arachnida), wie z. B. Mücken und Zecken, und betrifft insbesondere die Nutzung von Schwarzkümmelöl (Nigella sativa Linn.) zur topischen Anwendung am Menschen.

Die vorliegende Erfindung betrifft daher eine Zusammensetzung zur topischen Verabreichung zur Verwendung in einem Verfahren zur Abwehr von Insekten und/oder Spinnentieren, umfassend Schwarzkümmelöl als Wirkstoff sowie weiterhin übliche pharmazeutisch/kosmetisch akzeptable Zusatz- und/oder Hilfsstoffe als Basis, wobei vorzugsweise Naturkosmetik-konforme Rohstoffe in der erfindungsgemäßen Zusammensetzung enthalten sind. Erfindungsgemäß wird das reine, kaltgepresste Schwarzkümmelöl eingesetzt, gegebenenfalls in Kombination mit Andiroba-Öl.

### Stand der Technik

Schwarzkümmel (Nigella sativa Linn.) gehört zur Familie der Hahnenfußgewächse (Ranunculaceae). Seine Samen sowie das daraus gewonnene Öl oder Extrakte finden seit langem Anwendung als traditionelles Heilmittel. Den Inhaltsstoffen des Schwarzkümmels werden unter anderem entzündungshemmende, antioxidative, antimikrobielle und antiparasitäre Eigenschaften zugeschrieben.

Schwarzkümmelöl wird in der Naturheilkunde eingesetzt und zahlreiche Anbieter stellen Samen, Öl, Kapselpräparate oder Extrakte (Lipidfraktionen) davon bereit, z. B. als Arzneimittel zur Behandlung und Prophylaxe intraoraler Erkrankungen.

Darüber hinaus beschreibt die DE 103 08 346 A1 kosmetische und dermatologische Zubereitungen mit Extrakten aus Schwarzkümmel, welche beispielsweise zur Behandlung und Prophylaxe von UV-Schäden der Haut eingesetzt werden können. In der DE 20 2008 005 288 U1 wird die Verwendung von Schwarzkümmelöl zur Behandlung oder Prophylaxe entzündlicher Zahnerkrankungen und Erkrankungen der Mundschleimhaut beschrieben. In der DE 20 2005 007 603 U1 wird Schwarzkümmelöl in Kombination mit Arganöl in einer topisch zu verabreichenden Zusammensetzung offenbart, die den Zellregenerationsprozess und damit die Zellneubildung positiv beeinflussen soll. Eine Pflegesalbe zur Behandlung von trockener, stark beanspruchter und durch Strahlen geschädigter Haut, welche Schwarzkümmelöl ebenfalls in Kombination mit anderen Ölen offenbart, wird in der DE 202 09 721 U1 gelehrt. Darüber hinaus ist aber auch die orale Verabreichung von Schwarzkümmelöl bei verschiedenen Indikationen bekannt.

Das Dokument D2 (CARROLL J F ET AL, "Repellency to ticks (Acari: Ixodidae) of extracts of Nigella sativa (Ranunculaceae) and the anti-inflammatory DogsBestFriend (TM)", EXPERIMENTAL AND APPLIED ACAROLOGY, CHAPMAN & HALL, GB, Bd., Nr. 1, 9. Juli 2016 (2016-07-09), Seiten 89-97, XP036032963*)* beschreibt, dass eine topische Zusammensetzung mit einem ätherischen Schwarzkümmelextrakt eine zeckenabwehrende Wirkung bei Hunden hat.

Aus dem Dokument D7 *(*Anonymous: "Regensburger Schüler entdeckt wirksames Mittel gegen Zecken", 30. Mai 2014 (2014-05-30), XP055612237, Gefunden im Internet: URL: http://wissenschaft/er.de/news/item/4975-regensburger-schülerentdeckt-wirksames-mittel-gegenzecken?tmpl=component&print=1&jjj=1565602088630 *[gefunden am 2019-08-12])* kann der Fachmann entnehmen, dass auch oral verabreichtes Schwarzkümmelöl eine zeckenabwehrende Wirkung bei Hunden hat. Bei Versuchen wurde eine oberflächliche Anwendung auf Schweiß simuliert.

Topische Zusammensetzungen mit Schwarzkümmelöl, insbesondere mit nativem Schwarzkümmelöl, zur Abwehr von Insekten und/oder von Mücken und Zecken sind nicht vorbeschrieben.

Zu den bekannten Methoden zur Vorbeugung/Prophylaxe von Insektenstichen gehören neben mechanischen Mitteln, wie Gazefenster, Moskitonetze, dicht gewebte Kleidung, auch insektenvertreibende Mittel (Repellentien). Gegenwärtig werden hauptsächlich kommerzielle Produkte mit synthetischen Inhaltsstoffen, wie beispielsweise N,N-Diethyl-3-methylbenzamid (DEET), Dimethylphtalat (Palatinol M, DMP) sowie insbesondere 3-(N-n-Butyl-N-acetyl-amino)-propionsäureethylester, und para-Menthandiol als Repellentien zum Schutz vor Mücken, Zecken und Spinnen und dergleichen eingesetzt.

Nachteile dieser Repellentien sind, dass die enthaltenen chemischen Zusätze teilweise Allergien etc. verursachen können und auch keine Naturkosmetik-konformen Inhaltsstoffe sind. Die Verwendung solcher Repellentien ist aufgrund der Inhaltsstoffe häufig eingeschränkt, beispielsweise für Kinder, Schwangere und Stillende.

Es besteht daher ein Bedarf an natürlichen Mitteln zur Abwehr von Insekten und/oder Spinnentieren, welche auf DEET und andere synthetische Inhaltsstoffe verzichten, biologisch abbaubar sind und aus Pflanzen gewonnene Inhalts- und/oder Wirkstoffe enthalten.

Der Erfindung liegt deshalb die Aufgabe zugrunde, verbesserte Repellentien-Zusammensetzungen bereitzustellen, die auf der Basis von unbedenklichen pflanzlichen Rohstoffen zubereitet werden und die vorstehend genannten Nachtteile überwinden.

### Zusammenfassung/Besehreibunii der Erfindung

Es wurde erfindungsgemäß überraschender Weise erkannt, dass die obige Aufgabe, durch eine topische Zusammensetzung zur Verwendung in einem Verfahren gemäß Anspruch 1 gelöst wird. Die Zusammensetzung enthält Schwarzkümmelöl als Wirkstoff sowie einen oder mehrere übliche pharmazeutisch/kosmetisch akzeptable Zusatz- und/oder Hilfsstoffe, die die Basis bilden. Die erfindungsgemäßen Rezepturen enthalten vorzugsweise Naturkosmetik-konforme Rohstoffe und sind bevorzugt frei von Silikonen, Paraffinen, Parabenen, PEG, Mineralöl, synthetischen Polymeren, Glykol etc.. Bevorzugt sind vegane Rohstoffe zu verwenden. Als Wirkstoff wird das reine, kaltgepresste Schwarzkümmelöl gegebenenfalls in Kombination mit Andiroba-Öl verwendet, und zwar zum vorbeugenden Schutz vor Stichen und Bissen von Insekten, beispielsweise vonMücken, und/oder von Spinnentieren, beispielsweise von Zecken, d. h. es erfolgt keine Anwendung, im Sinne einer Behandlung von bereits vorhandenen Stichen und Bissen. Die Erfindung betrifft also gemäß einem Aspekt die topische Zusammensetzung zur Verwendung in einem Verfahren zum Vorbeugen von Stichen und/oder Bissen von Insekten und/oder von Spinnentieren. Ebenso umfasst ist gemäß einem weiteren Aspekt die Vorbeugung von durch diese Stiche oder Bisse übertragbaren Krankheiten.

Das native kaltgepresste Schwarzkümmelöl weist unter anderem einen hohen Gehalt an mehrfach ungesättigten Fettsäuren, wie vor allem der Linolensäure auf, aus der im menschlichen Körper durch das Enzym Delta-6-Desaturase die gamma-Linolensäure und die Arachidonsäure entsteht. Aus der gamma-Linolensäure, welche die Zellmembranen stabilisiert, entsteht Prostaglandin E1 (PGE1), welches wiederum stark entzündungshemmend ist und die Freisetzung allergischer Mediatoren hemmt. Ferner stabilisiert Schwarzkümmelöl auch die übersteigerte T-Zellfunktion des Allergikers und unterdrückt die pathologisch gesteigerte humorale Immunreaktion der B-Zellen. Zudem entsteht aus der Arachidonsäure Prostaglandin E2 (PGE2) was zusätzlich stark bronchienerweiternd wirkt.

Erfindungsgemäß wurde nun festgestellt, dass die topische Anwendung von Schwarzkümmelöl eine unerwartete Wirkung bei der Abwehr von Insekten und/oder Spinnentieren zeigt.

Erfindungsgemäß zeigte sich in einer Ausführungsform der Erfindung auch eine Kombination von Schwarzkümmelöl mit Andirobaöl (Carapa Guianensis) als gesteigert wirksam.

Die vorliegende Erfindung stellt daher eine topische Zusammensetzung zur Verwendung in einem Verfahren zur Abwehr von Insekten (Insecta) und/oder von Spinnentieren (Arachnida) bereit, wobei die Zusammensetzung Schwarzkümmelöl (Nigella sativa Linn.) als Wirkstoff sowie übliche pharmazeutisch/kosmetisch akzeptable Zusatz- und/oder Hilfsstoffe, die die Basis bilden, enthält.

Darüber hinaus beschreibt die vorliegende Erfindung in einer anderen Ausführungsform die Verwendung von Schwarzkümmelöl, gegebenenfalls auch in Kombination mit Andirobaöl (Carapa Guianensis), zur topischen Verabreichung und zwar im Besonderen zur Abwehr von Insekten und/oder Spinnentieren. Ebenso betrifft die Erfindung die Verwendung von Schwarzkümmelöl, gegebenenfalls auch in Kombination mit Andirobaöl (Carapa Guianensis), zum Vorbeugen von Stichen und/oder Bissen von Insekten und/oder von Spinnentieren. Ebenso umfasst ist gemäß einem weiteren Aspekt die Verwendung zur Vorbeugung von durch diese Stiche oder Bisse übertragbaren Krankheiten.

Ferner sind erfindungsgemäß umfasst Verfahren zum Vorbeugen von Stichen und/oder Bissen von Insekten und/oder von Spinnentieren sowie Verfahren zum Vorbeugen von durch diese Stiche oder Bisse übertragbaren Krankheiten. Diese Verfahren umfassen den Schritt einer topischen Applikation der hierin offenbarten Zusammensetzung.

Der topischen Zusammensetzung ist/sind ein oder mehrere übliche pharmazeutisch/kosmetisch akzeptable Zusatz- und/oder Hilfsstoffe zugesetzt, der/die die Basis bilden. Beispiele hierfür sind Stoffe aus der Gruppe, bestehend aus Emulgatoren, Detergentien, Schaumstabilisatoren, Verdickungsmitteln, Feuchthaltemitteln, wässerigen Trägern, ausgewählt aus Wasser, Alkohol, Wasser-Alkoholgemischen, Weichmachern, wie beispielsweise Glycerin oder Glycerin-Wassergemische; öligen Trägern, vorzugsweise ausgewählt aus natürlichen Ölen oder Neutralölen; Cremegrundlagen, ausgewählt aus Öl/Wasser- und Wasser/Öl-Grundlagen, Vaseline, Wachsen; Permeationsbeschleunigern, Entschäumungsmitteln, Konservierungsmitteln, Emollientien, Puffersubstanzen, Antioxidantien, insbesondere Rosmarinextrakt (Rosmarinus officinalis-Blätterextrakt), Geruchskorrigentien, Parfümen, Farbstoffen, Fettsäuren, quarternären Verbindungen, Moisturizern, Hautschutzstoffen, pflegenden Komponenten, Vitaminen, Lichtschutzfaktoren, anti-inflammatorisch wirkenden Substanzen, Filmbildnern, Antistatika, Fetten, Hydratisierer, Gelbildner und Konsistenzgeber. Vorzugsweise sind Naturkosmetik-konforme Rohstoffe in der Zusammensetzung enthalten, und die Zusammensetzung ist besonders bevorzugt frei von Silikonen, Paraffinen, Parabenen, PEG, Mineralöl, synthetischen Polymeren und/oder Glykolen.

Die vorstehend genannten Zusatz- und Hilfsstoffe können der verwendeten topischen Zusammensetzung einzeln oder in jeder beliebigen Kombination miteinander zugesetzt werden.

In den Unteransprüchen werden vorteilhafte Ausführungsformen der Erfindung beschrieben.

### Detaillierte Beschreibung der Erfindung

### Inhaltsstoffe:

### Wirkstoff:

Die erfindungsgemäße Zusammensetzung enthält als Wirkstoff Schwarzkümmelöl. Das Schwarzkümmelöl ist ein reines kaltgepresstes Öl, noch bevorzugter ein 100 % kaltgepresstes Schwarzkümmelöl aus kontrolliert biologischem Anbau.

Die erfindungsgemäßen Ausführungsformen weisen einen Anteil an Schwarzkümmelöl im Bereich von 15-35 Gew.-%, und beispielshaft von 25 Gew.-% auf.

Die Angabe Gew.-%, wie in der vorliegenden Patentanmeldung verwendet, bedeutet, sofern nichts anderes angegeben wird, dass der Anteil auf das Gesamtgewicht der Zusammensetzung bezogen ist.

Die Herstellung des Schwarzkümmelöls erfolgt durch eine kalte Pressung der sonnengereiften Samen, d. h. die Ausgangsstoffe werden lediglich in einem einzigen Arbeitsgang mithilfe einer Presse mechanisch ausgepresst und anschließend filtriert. Es findet keine Raffination oder ein Zusatz chemischer Stoffe statt.

Damit enthält das für die topische Zusammensetzung verwendete reine, kaltgepresste Öl keine chemischen Zusatzstoffe. Es zeichnet sich durch das komplette Spektrum der primären und sekundären Inhaltsstoffe der Schwarzkümmelsamen aus und ist eben nicht, wie im Stand der Technik in anderen Fällen vorbeschrieben, ein Extrakt des Schwarzkümmels, insbesondere kein alkoholischer Extrakt oder ein anderer organischer Lösungsmittel-Extrakt, wie beispielsweise ein Petrolether-Extrakt oder Hexan-Extrakt. Ebenso nicht erfindungsgemäß sind gemäß dieser Ausführungsform wässerige Extrakte, Dampfdestillate oder CO₂-Extrakte.

Das native kaltgepresste Öl ist, wie oben beschrieben, sehr reichhaltig und mannigfaltig, was die chemische Zusammensetzung angeht. Es weist im fetten Öl einen hohen Gehalt an mehrfach ungesättigten Fettsäuren, vor allem Linolensäure, auf, die im menschlichen Körper enzymatisch zur gamma-Linolensäure und zur Arachidonsäure umgesetzt wird. Aus der gamma-Linolensäure entsteht wiederum Prostaglandin E1, welches entzündungshemmend ist und die Freisetzung allergischer Mediatoren hemmt. Darüber hinaus sind zahlreiche Aminosäuren, Proteine und Kohlenhydrate im nativen Öl des Schwarzkümmels enthalten.

Darüber hinaus umfasst das native Öl aber auch ein breites Spektrum an sekundären pflanzlichen Inhaltsstoffen, wie beispielsweise Sterole, unter anderem Sitosterol, Cholesterol, Stigmastanol und Campesterol, aber auch Terpenoide, Tannine, Flavanoide, Glycoside, Alkaloide und Saponine. Die Zusammensetzung kann erntebedingten Schwankungen unterliegen, weshalb die Menge an Inhaltsstoffen variieren kann. Spezifikationsparameter für besonders geeignete Schwarzkümmel-Öle sind beispielsweise deren Brechungsindex, bevorzugt in einem Bereich von 1,3-1,5, deren relative Dichte, besonders bevorzugt in einem Bereich von 0,85-0,95 sowie das Vorhandensein und/oder der Gehalt an unterschiedlichen Fettsäuren. Erwähnenswert sind beispielsweise Ölsäure, vorzugsweise im Bereich von 15-35 Gew.-%, Linolsäure, vorzugsweise im Bereich von 48-70 Gew.-% oder Linolensäure, vorzugsweise bis maximal 1,5 Gew.-%.

Gemäß der vorliegenden Erfindung ist das native, kaltgepresste Schwarzkümmelöl, das allein wirksame Prinzip, d.h. es wird nicht in Kombination mit anderen Wirkstoffen, wie beispielsweise in Kombination mit ätherischen "Ölen", wie beispielsweise Lavendelöl, Citrusöl und/oder Orangenöl eingesetzt.

Gemäß einer weiteren Ausführungsform kann das Schwarzkümmelöl mit Andiroba-Öl kombiniert werden. Bei einer solchen Wirkstoffkombination kann der Anteil von Schwarzkümmelöl, bezogen auf die Gesamtzusammensetzung, im Bereich von vorzugsweise 15-25 Gew.-%, beispielsweise bei 20 Gew.-%, und der Anteil von Andiroba-Öl im Bereich von vorzugsweise 1-10 Gew.-%, noch mehr bevorzugt im Bereich von 2-8 Gew.%, beispielsweise bei 5 Gew.-%, liegen. Ebenso erfindungsgemäß umfasst sind solche Wirkstoffkombinationen, die, bezogen auf die Gesamtzusammensetzung, einen Anteil an Schwarzkümmelöl im Bereich von vorzugsweise 50-70 Gew.-%, beispielsweise bei 60 Gew.-%, aufweisen.

Wie bereits vorstehend erläutert, ist es besonders bevorzugt auch in solchen Wirkstoffkombinationen das native, vorzugsweise kaltgepresste Schwarzkümmelöl einzusetzen.

Bei dem gemäß diesen erfindungsgemäßen Ausführungsformen eingesetzten Andiroba-Öl handelt es sich vorzugsweise um kaltgepresstes Öl aus dem Samen des Andiroba-Baumes. Besonders bevorzugt ist dieses, sofern verfügbar, aus kontrolliert biologischem Anbau einzusetzen.

Bei den erfindungsgemäß in der topischen Zusammensetzung enthaltenen pharmazeutisch/kosmetisch akzeptablen Zusatz- und/oder Hilfsstoffen handelt es sich grundsätzlich um die üblichen, dem Fachmann bekannten Zusatz- und Hilfsstoffen, die in topischen Formulierungen eingesetzt werden können.

Die Zusatz- und/oder Hilfsstoffe umfassen im Besonderen:

### Pflanzliche Öle und Fette:

Als Zusatz- oder Hilfsstoff kann, gegebenenfalls auch in Kombination mit einem der anderen genannten Zusatz- und Hilfsstoffe, ein pflanzliches Öl oder ein Fett in der topischen Zusammensetzung enthalten sein.

Als pflanzliche Öle und Fette kommen grundsätzlich alle im Stand der Technik bekannten pflanzlichen Öle und Fette in Frage. Beispielsweise seien Kokosöl (INCI: Cocos nucifera-Öl), Aprikosenkernöl (INCI: Prunus Armeniaca (Apricot) Kernel Oil), Arganöl (INCI: Argania Spinosa Kernel Oil), Avocadoöl (INCI: Persea Gratissima (Avocado) Oil), Jojobaöl (INCI: Simmondsia Chinensis (Jojoba) Seed Oil), Macadamianussöl (INCI: Macadamia Ternifolia Seed Oil), Mandelöl (INCI: Prunus Amygdalus Dulcis (Sweet Almond) Oil), Olivenöl (INCI: Olea Europaea (Olive) Fruit Oil), Sesamöl (INCI: Sesamum Indicum (Sesame) Oil), Sojaöl (INCI: Glycine Soja (Soybean) Oil), Sonnenblumenöl (INCI: Helianthus Annuus (Sunflower) Seed Oil), Sheabutter (INCI: Butyrospermum Parkii (Shea) Butter) und/oder Kakaobutter (INCI: Theobroma Cacao (Cocoa) Seed Butter), genannt. Ganz besonders vorteilhaft ist das Öl aus den Samen der Sonnenblume (Helianthus annuus).

Die vorstehend genannten pflanzlichen Öle und Fette können einzeln oder in Mischungen davon eingesetzt werden. Sie sind besonders bevorzugt aus kontrolliert biologischem Anbau stammend.

Der Anteil an pflanzlichem Öl oder Fett oder einer Mischung pflanzlicher Öle und/oder Fette in der topischen Zusammensetzung beträgt vorzugsweise 1-15 Gew.-%, noch mehr bevorzugt 1-10 Gew.-% und ganz besonders bevorzugt 1-5 Gew.-%.

### Pflanzensäfte und -wässer:

Als Zusatz- oder Hilfsstoff kann, gegebenenfalls auch in Kombination mit einem der anderen genannten Zusatz- und Hilfsstoffe, ein Pflanzensaft oder ein Pflanzenwasser in der topischen Zusammensetzung enthalten sein. Als Pflanzensäfte und -wässer kommen grundsätzlich alle im Stand der Technik bekannten in Frage. Beispiele hierfür sind Aloe Vera Saft, Zitronenwasser und Orangenwasser.

Die dafür verwendeten Pflanzen stammen besonders bevorzugt aus kontrolliert biologischem Anbau.

Pflanzensäfte und -wässer können einzeln oder in Mischungen davon eingesetzt werden. Sie können zum Teil oder komplett das in der Zusammensetzung enthaltene Wasser ersetzen.

### Extrakte:

Als Zusatz- oder Hilfsstoff kann, gegebenenfalls auch in Kombination mit einem der anderen genannten Zusatz- und Hilfsstoffe, ein Extrakt in der topischen Zusammensetzung enthalten sein. Grundsätzliche kommen alle im Stand der Technik bekannten Extrakte in Frage. Besonders bevorzugt sind Pflanzenextrakte zu verwenden, wie beispielsweise Extrakte aus Grüntee (Camellia sinensis L.), Hamamelis (Hamamelis virginiana), Kamille (Matricaria recutita L.), Melisse (Melissa officinalis) oder Ringelblume (Calendula officinalis L.).

Die dafür verwendeten Pflanzen stammen besonders bevorzugt aus kontrolliert biologischem Anbau. Als Extraktionsmittel zur Herstellung der Extrakte werden üblicherweise besonders bevorzugt Wasser, Ethanol, Glycerin, Öle, Hexan und/oder CO₂ eingesetzt.

Die Extrakte können einzeln oder in beliebigen Mischungen davon eingesetzt werden.

### Pflanzenpulver:

Als Zusatz- oder Hilfsstoff kann, gegebenenfalls auch in Kombination mit einem der anderen genannten Zusatz- und Hilfsstoffe, ein Pflanzenpulver in der topischen Zusammensetzung enthalten sein. Grundsätzlich kommen alle im Stand der Technik bekannten Pflanzenpulver in Frage.

Die hierfür verwendeten Pflanzen stammen besonders bevorzugt aus kontrolliert biologischem Anbau. Die Pflanzenpulver können einzeln oder in Mischungen davon in der erfindungsgemäßen Zusammensetzung eingesetzt werden bzw. umfasst sein.

### Emulgatoren:

Ebenso kann/können der erfindungsgemäßen Zusammensetzung als Zusatz- oder Hilfsstoff ein oder mehrere Emulgatoren zugesetzt werden. Als Emulgator kommen grundsätzlich alle im Stand der Technik bekannten Emulgatoren in Frage. Emulgatoren können einzeln oder in beliebigen Mischungen von verschiedenen Emulgatoren eingesetzt werden. Vorzugsweise werden erfindungsgemäß als Emulgatoren Glycerylstearatcitrat, beispielsweise mit einem Anteil von 5 Gew.-%, Diisosteraroylpolyglyceryl-3-Dimer Dilinoleat, beispielsweise mit einem Anteil von 10 Gew.-%, Cetearylglucosid, beispielsweise mit einem Anteil von 2,00 Gew.-%, oder Kombinationen aus Glycerylstearatcitrat, beispielsweise mit einem Anteil von 5 Gew.-%, und Cetearylglucoside, beispielweise mit einem Anteil von 2,00 Gew.-%, eingesetzt.

Denkbar sind aber auch der Einsatz vonSucrosedistearat, Sucrosestearat, Natriumstearoylglutamat, Polyglyceryl-6-stearat, Polyglyceryl-6-behenat, Polyglyceryl-6-distearat und/oder Cetearylglucosid.

In einer bevorzugten Ausführungsform beträgt der maximale Anteil an Emulgator oder einer beliebigen Mischung von Emulgatoren 0,5-10 Gew.-%.

### Weichmacher/Emollient:

Als Zusatz- oder Hilfsstoff kann, gegebenenfalls auch in Kombinationen mit einem der anderen genannten Zusatz- und Hilfsstoffe, ein oder mehrere Weichmacher eingesetzt werden. Als Weichmacher kommen grundsätzlich alle im Stand der Technik bekannten Weichmacher in Frage. Weichmacher können einzeln oder in Mischungen von verschiedenen Weichmachern eingesetzt werden. Vorzugsweise werden erfindungsgemäß als Weichmacher eingesetzt Isoamyllaurat, Coco-Caprylate, Coco-Caprylate/Caprate, Dicaprylylcarbonate, Caprylyl-Caprylate/Caprate, Decyl-Oleate, Isopropyl-Myristate, Caprylic/Capric-Triglyceride, Cocoglyceride und/oder Myristyl Myristate. Ebenso können pflanzliche Öle als Weichmacher eingesetzt werden.

Ein besonders bevorzugter Weichmacher ist Isoamyllaurat.

In einer bevorzugten Ausführungsform beträgt der maximale Anteil an Weichmacher(n) 1-15 Gew.-%, vorzugsweise 1,5-10 Gew.-%, noch mehr bevorzugt 2-8 Gew.-%.

### Konsistenzgeber:

Als Zusatz- oder Hilfsstoff kann, gegebenenfalls auch in Kombinationen mit einem der anderen genannten Zusatz- und Hilfsstoffe, ein Konsistenzgeber eingesetzt werden. Konsistenzgeber erhöhen die Stabilität von Emulsionen und verbessern die Textur der Zusammensetzung. Als Konsistenzgeber kommen grundsätzlich alle im Stand der Technik bekannten Konsistenzgeber in Frage. Konsistenzgeber können einzeln oder in Mischungen von verschiedenen Konsistenzgebern eingesetzt werden. Beispiele für Konsistenzgeber sind Cetearylalkohol, Cetylalkohol, Stearylalkohol, Cetylpalmitat, Myristyl Myristate oder Wachse, wie beispielsweise Bienenwachs, Candelillawachs, Carnaubawachs oder Beerenwachs. Besonders bevorzugt wird Cetearylalkohol als Konsistenzgeber eingesetzt. Cetearylalkohol ist eine Mischung aus Cetylalkohol und Stearylalkohol.

Sofern vorhanden, beträgt in einer bevorzugten Ausführungsform der Anteil an Konsistenzgeber(n) 0,7-2 Gew.-%, noch mehr bevorzugt 1-1,8 Gew.-%.

### Antioxidationsmittel:

Als Zusatz- oder Hilfsstoff kann, gegebenenfalls auch in Kombinationen mit einem der anderen genannten Zusatz- und Hilfsstoffe, ein Antioxidationsmittel in der topischen Zusammensetzung enthalten sein. Antioxidationsmittel dienen beispielsweise zur Stabilisierung von fetten Ölen, ätherischen Ölen, sekundären Inhaltsstoffen, etc. Als Antioxidationsmittel kommen grundsätzlich alle im Stand der Technik bekannten Antioxidationsmittel in Frage. Antioxidationsmittel können einzeln oder in Mischungen davon eingesetzt werden. Beispiele für Antioxidationsmittel sind wasserlösliche Antioxidationsmittel, wie beispielsweise Vitamine, z. B. Ascorbinsäure und deren Derivate, Vitamin E und dessen Derivate sowie Vitamin A und dessen Derivate.

Erfindungsgemäß besonders bevorzugte Antioxidantien sind Rosmarinus Officinalis (Rosmarinblätterextrakt), Tocopherol, Helianthus Annuus (Sonnenblumensamenöl) oder beliebige Kombinationen davon. Diese können wiederum in Kombination mit anderen Antioxidantien, beispielsweise den vorstehend genannten Vitaminen, eingesetzt werden.

Die Herstellung von Rosmarin-Blattextrakt erfolgt vorzugsweise durch eine Hochdruckextraktion mit einer natürlichen Quellkohlensäure unter Zusatz einer geringen Ethanolmenge als Schleppmittel. Damit werden keine Rückstände von anorganischen Salzen und Schwermetallen, sowie keine vermehrungsfähigen Keime festgestellt. Durch mehrstufige Abscheidung wird das ätherische Öl weitgehend entfernt. Der dabei gewonnene Extrakt wird mit Sonnenblumenöl standardisiert.

Inhaltsstoffe des Rosmarinextraktes sind unter anderem 16-18 % antioxidativ wirksame Diterpenphenole mit mindestens 10 % Carnosolsäure und 1-4 % wasserdampfflüchtige Aromabestandteile. Der Wassergehalt beträgt vorzugsweise < 1 % und der Restgehalt an Ethanol ist vorzugsweise < 2 %. Darüber hinaus kann der eingesetzte Rosmarin-Blattextrakt Sonnenblumenöl (ökologisch) und Cuticularwachse enthalten.

Der erfindungsgemäß eingesetzte Rosmarinextrakt ist vorzugsweise ausschließlich aus dem genannten Rohstoff hergestellt, er enthält außer dem Sonnenblumenöl (ökologisch) keine Zusätze oder technischen Hilfsstoffe. Das Produkt ist zu 100 % natürlich und entspricht den Artikeln der EU-Aromenverordnung 1334/2008 für Aromaextrakte.

Der Anteil des Antioxidationsmittels (eine oder mehrere Verbindungen) in der Zusammensetzung beträgt vorzugsweise 0,01-0,5 Gew.-%, noch mehr bevorzugt 0,02-0,3 Gew.-%, beispielsweise 0,03 Gew.-%, bezogen auf das Gesamtgewicht der Zusammensetzung.

### Gelbildner/Stabilisator:

Die Zusammensetzung kann ferner einen oder mehrere Gelbildner oder Stabilisatoren umfassen. Diese sorgen für eine Stabilisierung und Festigkeit der Zusammensetzung, wenn diese beispielsweise als Creme oder Lotion formuliert ist. Als Gelbildner/Stabilisatoren kommen grundsätzlich alle im Stand der Technik bekannten in Frage.

Beispiele für Gelbildner sind Xanthan, Tapioca-Stärke, Guarkernmehl, Cellulose, Mannan und/oder Konjac Mannan. Xanthan ist erfindungsgemäß als Gelbildner bevorzugt.

Der Anteil an Gelbildner (einzeln oder in einer Mischung von Gelbildner) beträgt vorzugsweise 0,1-2 Gew.-%, noch mehr bevorzugt 0,2-1 Gew.-% und höchst bevorzugt 0,2-0,5 Gew.-%. Der Anteil kann beispielsweise 0,30 Gew.-% sein.

### Feuchthaltemittel:

Als Zusatz- oder Hilfsstoff kann, gegebenenfalls auch in Kombinationen mit einem der anderen genannten Zusatz- und Hilfsstoffe, ein Feuchthaltemittel in der topischen Zusammensetzung enthalten sein. Feuchthaltemittel binden bei der Herstellung zugesetztes Wasser und ein Feuchtigkeitsverlust bei der Lagerung wird vermieden. Als Feuchthaltemittel kommen grundsätzlich alle im Stand der Technik bekannten Feuchthaltemittel in Frage. Feuchthaltemittel können einzeln oder in Mischungen davon eingesetzt werden. Beispiele für Feuchthaltemittel, die in Hautpflegeprodukten eine Rolle spielen, sind Glycerin, Sorbit, 1,2-Propylenglycol, Xylit, Betain, Ectoin, Hyaluronsäure, Milchsäure, Harnstoff oder andere Polyalkohole.

Glycerin ist erfindungsgemäß als Feuchthaltemittel ganz besonders bevorzugt. Ebenso ist Sorbit (Sorbitol) oder eine Mischung aus Glycerin und Sorbit (Sorbitol) ein bevorzugtes Feuchthaltemittel.

Der Anteil des Feuchthaltemittels (eine oder mehrere Verbindungen) in der Zusammensetzung beträgt vorzugsweise 2-5 Gew.-%, beispielsweise 2,5 Gew.-%, bezogen auf das Gesamtgewicht der Zusammensetzung.

### Konservierungsmittel:

Die für diesen Zweck geeigneten und der Zusammensetzung optional zugesetzten Konservierungsmittel sind dem Fachmann bekannt und im Handel erhältlich. Als Konservierungsmittel ist Ethanol, Kaliumsorbat und/oder Natriumbenzoat gemäß einer Ausführungsform der vorliegenden Erfindung ganz besonders bevorzugt.

Der Anteil an Konservierungsmittel (eine oder mehrere Verbindungen) in der Zusammensetzung beträgt, vorzugsweise 2-15 Gew.-%, bevorzugt 4-12 Gew.-%, beispielsweise 5 Gew.-% oder 10 Gew.-%, bezogen auf das Gesamtgewicht der Zusammensetzung.

### Pflegende Komponenten:

Geeignete Substanzen und Substanzgemische, die der topischen Zusammensetzung als pflegende Komponente zugesetzt werden können, sind dem Fachmann wohl bekannt und können im Handel bezogen werden. Ganz besonders bevorzugt ist insbesondere Bisabolol.

Bisabolol ist ein entzündungshemmender Stoff, der im ätherischen Öl der Kamille vorkommt. Bisabolol kann aber auch synthetisch hergestellt werden. Gemäß einer Ausführungsform wird in der Zusammensetzung die isolierte Substanz und nicht Kamillenextrakt verwendet.

Der Anteil an pflegender Komponente (eine oder mehrere Verbindungen) in der Zusammensetzung beträgt vorzugsweise 0,02-1 Gew.-%, noch mehr bevorzugt 0,03-0,08 Gew.-%, beispielsweise 0,05 Gew.-%, bezogen auf das Gesamtgewicht der Zusammensetzung.

### Vitamine:

Als Zusatz- oder Hilfsstoff kann, gegebenenfalls auch in Kombination mit einem der anderen genannten Zusatz- und Hilfsstoffe, ein Vitamin oder mehrere Vitamine in der topischen Zusammensetzung enthalten sein.

Als Vitamine kommen grundsätzlich alle im Stand der Technik bekannten Vitamine in Frage, wie beispielsweise D-Panthenol, Nicotinamid, Vitamin C (Ascorbinsäure), Vitamin E (Tocopherol).

Vitamin können einzeln oder in Mischungen davon eingesetzt werden.

### Aminosäuren und Peptide:

Als Zusatz- oder Hilfsstoff kann, gegebenenfalls auch in Kombination mit einem der anderen genannten Zusatz- und Hilfsstoffe, eine Aminosäure oder ein Peptid in der topischen Zusammensetzung enthalten sein.

Als Aminosäuren und Peptide kommen grundsätzlich alle im Stand der Technik bekannten in Frage wie beispielsweise Glycin, Prolin, Lysin, Valin, Methionin, Cystein, Arginin und/oder Kollagenpeptide.

Aminosäuren und Peptide können einzeln oder in Mischungen davon eingesetzt werden.

### Mineralstoffe und Salze:

Als Zusatz- oder Hilfsstoff kann, gegebenenfalls auch in Kombination mit einem der anderen genannten Zusatz- und Hilfsstoffe, ein Mineralstoff oder ein Salz in der topischen Zusammensetzung enthalten sein.

Als Mineralstoffe und Salze kommen grundsätzlich alle im Stand der Technik bekannten in Frage. Denkbar sind beispielsweise Chloride, Sulfate, Carbonate von Magnesium, Natrium, Kalium und/oder Calcium.

Mineralstoffe und Salze können einzeln oder in Mischungen davon eingesetzt werden.

### Parfüm:

Als Zusatz- oder Hilfsstoff kann, gegebenenfalls auch in Kombination mit einem der anderen Hilfs- oder Zusatzstoffe, Parfüm, besonders bevorzugt natürliches Parfüm, in der erfindungsgemäßen Zusammensetzung umfasst sein. Ebenso sind Mischungen davon denkbar.

Der Anteil liegt vorzugsweise im Bereich von 0,1-2,5 Gew.-%, noch mehr bevorzugt im Bereich von 0,3-1,5 Gew.-%, beispielsweise bei 0,75 Gew.%.

### Ätherische Öle:

Als Zusatz- oder Hilfsstoff kann, gegebenenfalls auch in Kombination mit einem der anderen genannten Zusatz- und Hilfsstoffe, ein ätherisches Öl in der topischen Zusammensetzung enthalten sein.

Ätherische Öle sind flüchtige, fettlösliche, duftende Stoffwechsel-Produkte von Pflanzen, die in verschiedenen Verfahren gewonnen werden. Zu den typischen Gewinnungsverfahren zählen Wasserdampfdestillation, Pressung oder Extraktion mit beispielsweise Alkohol, Petrolether, Hexan, CO₂. Als ätherische Öle kommen grundsätzlich alle im Stand der Technik bekannten ätherischen Öle in Frage.

In der erfindungsgemäßen Zusammensetzung dienen ätherische Öle der Parfumierung und/oder der Antioxidation, weshalb hinsichtlich der bevorzugten Gew.-%-Anteile auf die vorstehende Offenbarung verwiesen werden kann.

Ätherische Öle können einzeln oder in Mischungen davon eingesetzt werden

### Erden:

Als Zusatz- oder Hilfsstoff kann, gegebenenfalls auch in Kombination mit einem der anderen genannten Zusatz- und Hilfsstoffe, eine Erde in der topischen Zusammensetzung enthalten sein.

Grundlage aller Erden ist Löss. Er besteht primär aus Quarz (Sand ca. 40-70 %), Tonerde-Silikaten (Feldspat, Glimmer ca. 10-30 %), Kalk (ca. 10-30 %), Mineralien und Spurenelementen. Als Erden kommen grundsätzlich alle im Stand der Technik bekannten Erden in Frage. Genannt seien beispielhaft grüne Mineralerde (Illite), Heilerde, Lavaerde, farbige Tonerden und Kaolin.

Erden können einzeln oder in Mischungen davon eingesetzt werden.

### Farbadditive/Pigmente:

Als Zusatz- oder Hilfsstoff kann, gegebenenfalls auch in Kombination mit einem der anderen genannten Zusatz- und Hilfsstoffe, ein Farbadditiv oder ein Pigment in der topischen Zusammensetzung enthalten sein.

Als Farbadditive und Pigmente kommen grundsätzliche alle im Stand der Technik bekannten in Frage, beispielsweise Mineralien wie Mica, Pflanzenfarbstoffe wie Chlorophyll, Betanin, Carotinoide, Flavonoide, Anthocyane, Ultramarinblau und/oder Azulen.

Farbadditive und Pigmente können einzeln oder in Mischungen davon eingesetzt werden.

### UV-Stabilisatoren/Lichtschutzpigmente:

Die erfindungsgemäße Zusammensetzung kann optional in einer weiteren Ausführungsform zusätzlich zu einem oder mehreren der hierin genannten Hilfs- oder Zusatzstoffe, mindestens eine UV-A- und/oder UV-B- Filtersubstanz enthalten.

Geeignete UV-Filtersubstanzen sind dem Fachmann bekannt. Beispielhaft genannt seien:
Homomethylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxyzimtsäure, INCI: Octyl Methoxycinnamate) und 4-Methoxyzimtsäureisopentylester (Isopentyl-4-methoxycinnamat, INCI: Isoamyl p-Methoxycinnamat), 3-(4-(2,2-bis Ethoxycarbonylvinyl)-phenoxy)propenyl)-methoxysiloxan/dimethylsiloxan-Copolymer, welches beispielsweise unter der Handelsbezeichnung Parsol bei Hoffmann La Roche erhältlich ist, Titandioxid (INCI: Titanium Dioxide) und Zinkoxid (INCI: Zinc Oxide).

### Schaumbildner und Schaumstabilisatoren:

Für den Fall, dass die Zusammensetzung als Schaum, Duschöl, Badezusatz, Seife formuliert ist, kann diese optional auch einen oder mehrere Schaumbildner und/oder Schaumstabilisatoren enthalten. Beispiele hierfür sind Decylglucosid (INCI: Decyl Glucoside), Kokosbetain (INCI: Cocamidopropyl Betaine) und/oder Kokosglucosid (INCI: Coco Glucoside).

### Wasser:

Weiterhin kann die topische Zusammensetzung auch Wasser enthalten. Der Anteil des Wasser, vorzugsweise von Wasser welches für kosmetische oder pharmazeutische Formulierungen geeignet ist, beträgt in den einzelnen Rezepturen immer den Rest auf 100 Gew.-% (Ad. 100 %), bezogen auf die Gesamtzusammensetzung.

Alle Bestandteile der pharmazeutischen/kosmetischen Zusammensetzung sind in einem Reinheitsgrad, der für topisch zu applizierende Zusammensetzungen geeignet und notwendig ist. Gemäß einer weiteren bevorzugten Ausführungsform der vorliegenden Erfindung sind in der Zusammensetzung vorzugsweise Naturkosmetik-konforme Rohstoffe umfasst. Weiterhin ist bevorzugt, dass die Zusammensetzungen frei von Silikonen, Paraffinen, Parabenen, PEG, Mineralöl, synthetischen Polymeren und/oder Glycolen, etc. sind. Nochmehr bevorzugt sind sie zusätzlich vegan.

Werden für bestimmte Hilfs- oder Zusatzstoffe hierin keine Angaben zu deren Gew.-%-Anteil gemacht, bedeutet dies, dass der Fachmann die bevorzugten Bereiche kennt oder einfach ermitteln kann.

Ebenfalls im Umfang der vorliegenden Erfindung mit umfasst sind solche Zusammensetzungen, die, mit Ausnahme von üblichen Verunreinigungen, nur aus den vorstehend genannten wesentlichen, und gegebenenfalls einer oder mehreren der optionalen Komponenten, bestehen.

Ganz besonders bevorzugt umfasst die topische Zusammensetzung den Wirkstoff Schwarzkümmelöl, gegebenenfalls in einer Kombination mit Andiroba-Öl, und als Hilfs- oder Zusatzstoffe einen oder mehrere Emulgatoren, vorzugsweise Glycerylstearatcitrat, Diisostearoylpolyglyceryl-3-Dimer-Dilinoleat und/oder Cetearylglucosid, einen oder mehrere Weichmacher und/oder ein oder mehrere Feuchthaltemittel, vorzugsweise Glycerin, ein oder mehrere Antioxidantien, vorzugsweise Rosmarin-Blätterextrakt, Tocopherol und/oder Sonnenblumenöl, einen oder mehrere Gelbildner, vorzugsweise Xanthan, ein oder mehrere Konservierungsmittel, vorzugsweise Ethanol, eine oder mehrerer pflegende Komponenten, Parfüm und als Rest Wasser. Die Zusammensetzung kann ferner einen oder mehrere Konsistenzgeber, vorzugsweise Cetearylalkohol, enthalten. Hinsichtlich der Gew.-%-Anteil der einzelnen Komponenten wird auf die vorstehenden Aufführungen zu den einzelnen Inhaltsstoffen verwiesen, auf die ausdrücklich Bezug genommen wird.

Gemäß einer weiteren bevorzugten Ausführung besteht die topische Zusammensetzung aus den im vorstehenden Abschnitt genannten Inhaltsstoffen, vorzugsweise in den genannten Gew.-%-Anteilen.

### Formulierungen

Die erfindungsgemäßen Zusammensetzungen sind zur topischen Applikation auf die Haut und/oder die Haare formuliert. Nicht umfasst sind Nahrungsergänzungsmittel, also Zusammensetzungen für eine orale Applikation.

Die Zusammensetzungen im Sinne der vorliegenden Erfindung können neben Wirkstoff und ggf. einer oder mehrerer Ölphasen zusätzlich noch eine oder mehrere Wasserphasen aufweisen. Beispielsweise können erfindungsgemäße Zusammensetzungen in Form von Wasser-in-Öl-Emulsionen, Öl-in-Wasser-Emulsionen, Wasser-in-Öl-in-Wasser-Emulsionen oder Öl-in-Wasser-in-Öl-Emulsionen, Lösungen sowie Salben, insbesondere lipophile, hydrophile und wasseraufnehmende Salben vorliegen.

Der oder die Wirkstoff(e) können auch in mikroverkapselter Form oder als Liposomen vorliegen.

Eine Zusammensetzung zur topischen Applikation auf die Haut in Form einer Lotion, vorzugsweise einer Körperlotion, einer Milch, einer Creme, beispielsweise einer Gesichts- und/oder Körpercreme, eines Gels, vorzugsweise eines Öl-Gels oder eines Liposomen-Gels, oder eines Serums ist besonders bevorzugt.

Ebenso bevorzugt kann die Zusammensetzung als Duschöl, Badezusatz, Seife oder Schaum formuliert sein. Geeignete Formulierungen und Hilfsstoffe für Duschöle, Badezusätze und Seifen sind dem Fachmann bekannt. Schäume im Sinne der vorliegenden Erfindung sind selbstschäumende, schaumförmige, nachschäumende oder schäumbare Zusammensetzungen. Geeignete Emulgatoren und Emulgatorsysteme für die Zubereitung von Schäumen sind dem Fachmann bekannt. Schaumverstärker und Schaumstabilisatoren, die besonders bevorzugt sind, sind vorstehend genannt.

Ferner kann die erfindungsgemäße Zusammensetzung zur Applikation auf das Haar oder Fell, beispielsweise in Form eines Haarshampoos, einer Haarspülung, eines Haargels, eines Haarsprays oder eines Haarlacks formuliert sein.

Ebenso mit umfasst sind sprühbare Emulsionen, wie beispielsweise eine sprühbare Körper- oder Haarmilch, Deo-Mittel-haltige W/O-Emulsionen, oder Deos.

Die erfindungsgemäßen Zusammensetzungen können auch als Mikroemulsion formuliert sein. Im Sinne der vorliegenden Erfindung sind Mikroemulsionen solche, die einen Tröpfchen-Durchmesser im Bereich von etwa 50 bis etwa 500 nm aufweisen. Im Fall von Mikroemulsionen sind sprühbare Mikroemulsionen besonders bevorzugt. Mikroemulsionen sind besonders vorteilhaft, weil der Wirkstoff oder die Wirkstoffe in wesentlich feinerer disperser Form vorliegen und damit eine bessere Verteilung auf der Körperoberfläche bewirkt werden kann.

Die erfindungsgemäßen Zusammensetzungen können aber auch als Stifte, beispielsweise Roll-On-Sticks formuliert sein und als solche eingesetzt werden. Solche Stifte können wasserfreie Fettmischungen aus festen oder halbfesten Wachsen und flüssigen Ölen sein, wobei hochgereinigte Paraffinöle und -wachse die Stiftgrundmasse darstellen. Die üblichen Grundstoffe für solche Stifte, wie beispielsweise Bienenwachs, Carnaubawachs, Candelillawachs, Beerenwachs, Sheabutter und/oder Lanolin sind dem Fachmann bekannt. Darüber hinaus können die Stifte auch wasserhaltige Zusammensetzungen sein und beispielsweise als W/O-Emulsion vorliegen.

### Vorteile der erfindungsgemäßen Zusammensetzung;

Die Formulierungen der vorliegenden Erfindung, nämlich die Emulsionen, zeichnen sich dadurch vorteilhaft aus, dass sie leicht aufzutragen sind und gut und schnell in die Haut einziehen. Damit hat der Nutzer beim und nach dem Auftragen kein klebriges Gefühl auf der Haut, was insbesondere in den Sommermonaten von großem Vorteil ist. Darüber hinaus weisen die Zusammensetzungen eine angenehme Haptik auf, haben ein angenehmes und leichtes Hautgefühl und weisen zusätzlich einen pflegenden Effekt auf.

Der Wirkstoff wird bevorzugt entsprechend stabilisiert und somit vor einem oxidativem Abbau geschützt. Damit wird das Öl nicht ranzig und Hautreizungen werden vermieden.

### Anwendung:

Zur Anwendung werden die topischen Zusammensetzungen in der für Kosmetika und die Formulierung üblichen Weise auf die Haut und/oder die Haare in ausreichender Menge aufgebracht.

Die Einwirkzeit bei allen Formulierungen auf der Haut beträgt vorzugweise mindestens 10 Sekunden, vorzugsweise mindestens 20 Sekunden, noch mehr bevorzugt mindestens 30 Sekunden und ganz besonders bevorzugt mindestens 40 Sekunden. Weiterhin kann es beispielsweise bei zur Körperreinigung formulierten abwaschbaren Zusammensetzungen, wie Duschgels, Badezusätzen, Shampoos etc., bevorzugt sein, dass die maximale Einwirkzeit der erfindungsgemäßen Zusammensetzung auf der Haut 30 Minuten, vorzugsweise 20 Minuten, 10 Minuten oder 8 Minuten, noch mehr bevorzugt 6 Minuten und ganz besonders bevorzugt maximal 4 Minuten beträgt.

Nach der Einwirkzeit werden die abwaschbaren Formulierungen vorzugsweise zumindest teilweise abgewaschen oder andersartig entfernt. Die auf der Haut verbleibenden Bestandteile der erfindungsgemäßen Zusammensetzung verhindern Insektenstiche/ -bisse oder Zeckenbisse. Je nach Zusammensetzung des Mittels können hier auch hautpflegende Aspekte zum Tragen kommen.

Es hat sich überraschenderweise gezeigt, dass durch die Anwendung der erfindungsgemäßen Zusammensetzung ein wirksamer Schutz vor Insektenstichen und Zeckenbissen und der durch sie übertragenen Krankheiten und/oder Krankheitserreger erreicht werden kann.

So sind Zecken beispielsweise Überträger von Lyme-Borreliose, Babesiose, Anaplasmose oder FSME (Hirnhautentzündung).

Mücken können beispielsweise Viruskrankheiten übertragen, die unter anderem Hautausschläge oder chronische Gelenkschmerzen hervorrufen können. Ebenso können durch Mückenstiche Malaria, Gelbfieber, Dengue-Fieber, das Zika-Virus, das Chikungunya-Virus oder das West-Nil-Fiber übertragen.

### Beispiele (erfindungsgemäß)

Im Folgenden wird die Erfindung anhand von einer beispielhaften Formulierung und Anwendung näher erläutert.

Beispiel (Emulsion) mit 25 Gew.-%: Schwarzkümmelöl

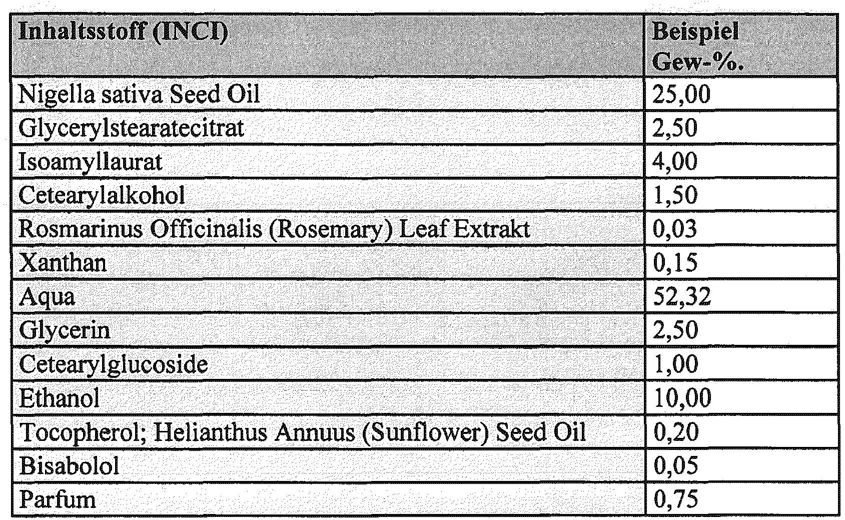

## Patentansprüche

1. Topische Zusammensetzung zur Verwendung in einem Verfahren zum Vorbeugen von Stichen und/oder Bissen von Insekten (Insecta) und/oder von Spinnentieren (Arachnida) beim Menschen und/oder zum Vorbeugen von durch diese Stiche und/oder Bisse übertragbaren Krankheiten, enthaltend natives, kalt gepresstes Schwarzkümmelöl (Nigella sativa Linn.) als Wirkstoff sowie einen oder mehrere übliche pharmazeutisch/kosmetisch akzeptable Zusatz- oder Hilfsstoffe, wobei
die Zusammensetzung eine Emulsion ist, enthaltend (a) eine das Schwarzkümmelöl enthaltende Ölphase und (b) eine Wasserphase, und wobei die Zusammensetzung umfasst:
15 bis 35 Gew.-% Schwarzkümmelöl,
0 bis 25 Gew.-% eines oder mehrerer weiterer Öle auf pflanzlicher Basis, insbesondere Andirobaöl und/oder Kokosöl,
0,5 bis 10 Gew.-% Emulgatoren,
0 bis 15 Gew.-%, vorzugsweise 1 bis 15 Gew.-% Emollient,
0,7 bis 2 Gew.-% Konsistenzgeber,
0,002 bis 0,5 Gew.-% Antioxidationsmittel,
0,15 bis 2 Gew.-% Gelbildner/Stabilisator,
0 bis 5 Gew.-%, vorzugsweise 2 bis 5 Gew.-% Feuchthaltemittel,
5 bis 15 Gew.-% Konservierungsmittel, insbesondere Ethanol,
0,005 bis 1 Gew.-% pflegende Komponenten, insbesondere Bisabolol,
0,1 bis 2,5 Gew.-% Parfüm, und
Wasser ad 100 Gew.-%.

2. Topische Zusammensetzung zur Verwendung gemäß Anspruch 1, **dadurch gekennzeichnet, dass** die Ölphase der Zusammensetzung weiterhin wenigstens ein Öl auf pflanzlicher Basis, ausgewählt aus der Gruppe bestehend aus Sonnenblumenöl, Kokosöl (Cocos Nucifera), Andirobaöl (Carapa Guianensis) und Mischungen davon enthält.

3. Topische Zusammensetzung zur Verwendung gemäß Anspruch 2, **dadurch gekennzeichnet, dass** der Anteil des weiteren pflanzlichen Öls oder der weiteren pflanzlichen Öle 0,1 bis 25 Gew.%, bezogen auf die Gesamtzusammensetzung, beträgt.

4. Topische Zusammensetzung zur Verwendung gemäß einem der Ansprüche 1 bis 3, **dadurch gekennzeichnet, dass** die Zusammensetzung eine Öl-in-Wasser-Emulsion und insbesondere eine Lotion oder eine Creme ist.

5. Topische Zusammensetzung zur Verwendung gemäß einem der vorhergehenden Ansprüche zum Vorbeugen von Insektenstichen und Zeckenbissen und der durch sie übertragenen Krankheiten.

## Claims

1. A topical composition for use in a method of preventing stings and/or bites of insects (Insecta) and/or arachnids (Arachnida) in humans and/or preventing diseases transmissible by said stings and/or bites, comprising native cold-pressed black cumin oil (Nigella sativa Linn.) as an active ingredient and one or more conventional pharmaceutically/cosmetically acceptable additives or adjuvants, wherein
the composition is an emulsion containing (a) an oil phase containing the black cumin oil and (b) a water phase, and wherein the composition comprises:
15 to 35% by weight of black cumin oil,
0 to 25% by weight of one or more other vegetable-based oils, in particular andiroba oil and/or coconut oil,
0.5 to 10% by weight of emulsifiers,
0 to 15% by weight, preferably 1 to 15% by weight, of emollient,
0.7 to 2 % by weight consistency enhancer,
0.002 to 0.5 % by weight antioxidant,
0.15 to 2 % by weight gel former/stabiliser,
0 to 5 % by weight, preferably 2 to 5 wt.% humectant,
5 to 15 % by weight, preservative agent, in particular ethanol,
0.005 to 1% by weight of nourishing components, in particular bisabolol,
0.1 to 2.5% by weight perfume, and
water to 100% by weight.

2. Topical composition for use according to claim 1, **characterised in that** the oil phase of the composition further comprises at least one vegetable-based oil selected from the group consisting of sunflower oil, coconut oil (Cocos Nucifera), andiroba oil (Carapa Guianensis) and mixtures thereof.

3. Topical composition for use according to claim 2, **characterised in that** the proportion of the further vegetable oil or oils is from 0.1 to 25% by weight of the total composition.

4. Topical composition for use according to any one of claims 1 to 3, **characterized in that** the composition is an oil-in-water emulsion and in particular a lotion or a cream.

5. Topical composition for use according to any one of the preceding claims for preventing insect stings and tick bites and the diseases transmitted by them.

## Revendications

1. Composition topique, destinée à l'utilisation dans un procédé de prévention de piqûres et/ou de morsures d'insectes (Insecta) et/ou d'arachnides (Arachnida) chez l'individu et/ou de prévention de pathologies transmissibles par lesdites piqûres et/ou morsures, contenant en tant que principe actif de l'huile de nigelle native, pressée à froid (Nigella sativa Linn.), ainsi qu'un ou plusieurs additifs ou excipients habituels, acceptables du point de vue pharmaceutique/cosmétique,
la composition étant une émulsion, contenant (a) une phase huileuse contenant l'huile de nigelle et (b) une phase aqueuse, et la composition comprenant :
de 15 à 35 % en poids d'huile de nigelle,
de 0 à 25 % en poids d'une ou de plusieurs autres huiles à base végétale, notamment d'huile d'andiroba et/ou d'huile de coco,
de 0,5 à 10 % en poids d'émulsifiants,
de 0 à 15 % en poids, de préférence de 1 à 15 % en poids d'émollient,
de 0,7 à 2 % en poids d'agent texturant,
de 0,002 à 0,5 % en poids d'agent antioxydant,
de 0,15 à 2 % en poids de gélifiant/de stabilisateur,
de 0 à 5 % en poids, de préférence de 2 à 5 % en poids d'humectant,
de 5 à 15 % en poids d'agent conservateur, notamment d'éthanol,
de 0,005 à 1 % en poids de composants de soin, notamment de de bisabolol,
de 0,1 à 2,5 % en poids de parfum, et
de l'eau ad 100 % en poids.

2. Composition topique destinée à l'utilisation selon la revendication 1, **caractérisée en ce que** la phase huileuse de la composition contient par ailleurs au moins une huile à base végétale, sélectionnée dans le groupe comprenant l'huile de tournesol, l'huile de coco (Cocos Nucifera), l'huile d'andiroba (Carapa Guianensis) et des mélanges de ces dernières.

3. Composition topique destinée à l'utilisation selon la revendication 2, **caractérisée en ce que** la part de l'huile végétale additionnelle ou des huiles végétales additionnelles est de 0,1 à 25 % en poids, rapportés à la composition totale.

4. Composition topique destinée à l'utilisation selon l'une quelconque des revendications 1 à 3, **caractérisée en ce que** la composition est une émulsion huile en eau et notamment une lotion ou une crème.

5. Composition topique destinée à l'utilisation selon l'une quelconque des revendications précédentes, pour la prévention de piqûres d'insectes et de morsures de tiques et des pathologies qu'elles transmettent.
